# EUROPEAN PATENT SPECIFICATION

(11) **EP 1 603 892 B1**
(45) Date of publication and mention of the grant of the patent: **05.10.2011**
(21) Application number: 04715057.8
(22) Date of filing: 26.02.2004
(51) Int. Cl.: C07D 301/10

(54) **A METHOD OF MANUFACTURING ETHYLENE OXIDE**
VERFAHREN ZUR HERSTELLUNG VON ETHYLENOXID
PROCEDE DE FABRICATION D'OXYDE D'ETHYLENE

(30) Priority: 28.02.2003 US 451162 P
(43) Date of publication of application: 14.12.2005
(73) Proprietor: Shell Internationale Research Maatschappij B.V., 2596 HR Den Haag (NL)
(72) Inventor: EVANS, Wayne, Errol, Richmond, Texas 77469 (US); MATUSZ, Marek, Houston, Texas 77084 (US); TE RAA, Arend, Jan, 2514 AB Den Haag (NL)
(86) International application number: PCT/US2004/005744
(87) International publication number: WO 2004/078736

(56) References cited:
- EP-A- 0 425 020
- EP-A- 0 480 539
- WO-A-01/96324
- CA-C- 1 286 689
- GB-A- 2 161 480
- US-A- 4 822 900

## Description

This invention relates to the efficient manufacture of ethylene oxide by the partial oxidation of ethylene with oxygen using a high selectivity epoxidation catalyst.

In recent years new highly selective ethylene epoxidation catalysts have been developed that provide for selectivity benefits over conventional high activity epoxidation catalysts. Such high selectivity catalysts are known from U.S. Patents 4,761,394 and 4,766,105. However, the high selectivity catalysts employ higher reaction temperatures than do the high activity catalysts for a given ethylene oxide yield, and they exhibit a greater rate of catalyst deactivation than conventional high activity epoxidation catalysts.

It is, therefore, desirable to find a way to take advantage of the selectivity benefits from using a high selectivity epoxidation catalyst in the manufacture of ethylene oxide without incurring such associated negative effects as indicated hereinbefore.

In accordance with the invention, a method is provided of manufacturing ethylene oxide, said method comprises:
charging a reactor feed comprising ethylene, oxygen, and a specific concentration of carbon dioxide to an epoxidation reaction zone containing a high selectivity epoxidation catalyst and operated under epoxidation reaction conditions;
yielding from said epoxidation reaction zone an epoxidation reactor effluent;
charging at least a portion of said epoxidation reactor effluent to an ethylene oxide absorber means for separating said at least a portion of said epoxidation reactor effluent into a gaseous overhead stream and an ethylene oxide stream;
dividing said gaseous overhead stream into a split portion stream, if any, and a remaining portion stream wherein said remaining portion stream is at least 40 percent upwardly to 100 percent of said gaseous overhead stream;
charging said remaining portion stream as a carbon dioxide containing feed gas to a carbon dioxide removal system means for separating said carbon dioxide containing feed gas into a carbon dioxide depleted gas stream and a carbon dioxide gas stream; and
combining at least a portion of said carbon dioxide depleted gas stream with oxygen and ethylene to thereby form said reactor feed.

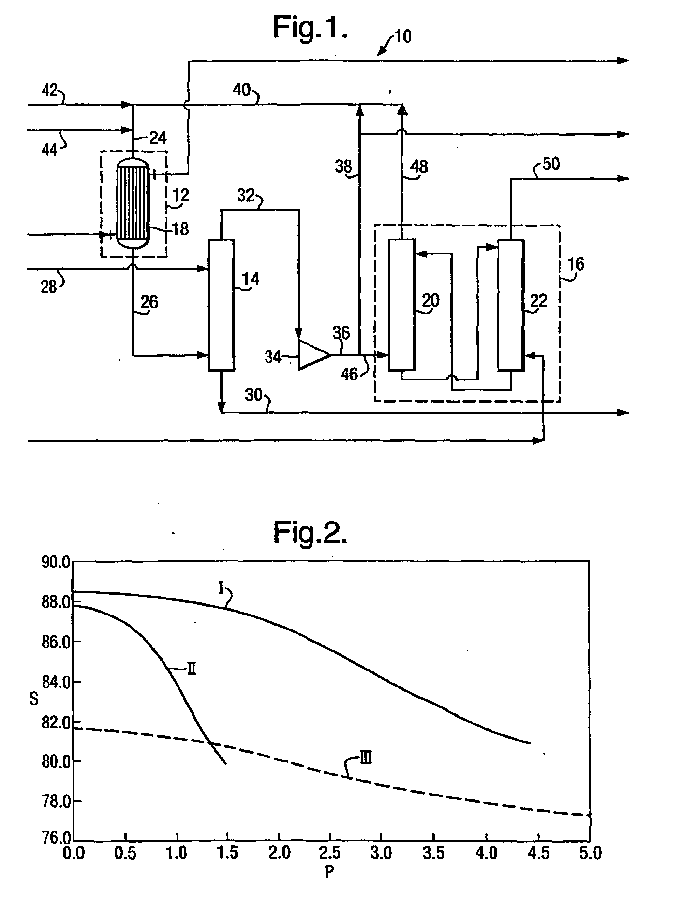

FIG. 1 is a process flow schematic representing an ethylene oxide manufacturing process that includes certain of the features of the inventive method.

FIG. 2 demonstrates the improvement in catalytic life and selectivity of a high selectivity catalyst with plots of catalyst selectivity ("S", in %), at a given work rate, as a function of cumulative ethylene oxide production ("P", in kton/m³) for the inventive use of a high selectivity epoxidation catalyst ("I") as compared to the conventional use of a high selectivity epoxidation catalyst ("II") and the conventional use of a high activity catalyst ("III");

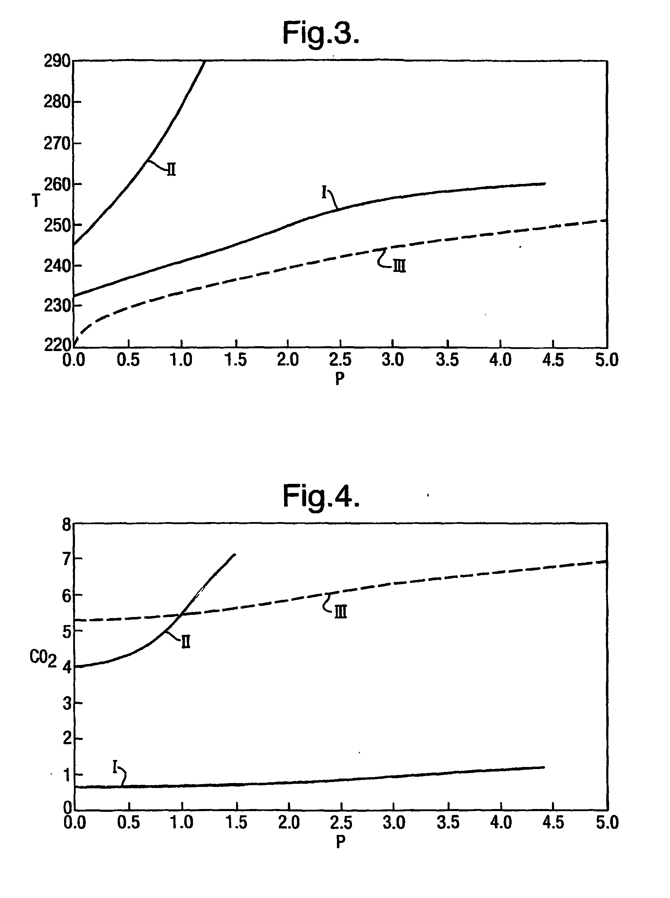

FIG. 3 demonstrates the improvement in catalytic life and reaction temperature with plots of reactor coolant temperature ("T", in °C) as a function of cumulative ethylene oxide production ("P", in kton/m³) for the inventive use of a high selectivity epoxidation catalyst ("I") and as compared to the conventional use of high selectivity epoxidation catalyst ("II") and the conventional use of a high activity catalyst ("III"); and

FIG. 4 presents plots of the reactor inlet concentration of carbon dioxide ("CO₂" in mole %) as a function of the cumulative ethylene oxide production ("P", in kton/m³) corresponding to the values for selectivity and reactor coolant temperature presented in FIG. 2 and FIG. 3.

The ethylene oxide manufacturing system of the inventive method comprises an epoxidation reactor system, an ethylene oxide recovery system, and a carbon dioxide removal system that are operatively connected to each other so as to provide for the partial oxidation of ethylene with oxygen to yield ethylene oxide and the recovery of an ethylene oxide product. Carbon dioxide is an unwanted by-product of the epoxidation reaction and it is usually removed from the ethylene oxide manufacturing system at a rate that approximates its production in order to prevent its buildup in the system.

It has been discovered that in the use of high selectivity epoxidation catalysts in the manufacture of ethylene oxide by the partial oxidation of ethylene with oxygen at constant conversion or work rate great improvement in the useful life of the catalyst and other benefits are obtainable by altering the composition of the typical epoxidation reactor feed. In an ethylene oxide process a typical epoxidation reactor feed generally comprises ethylene, oxygen and a concentration of carbon dioxide with the concentration in most cases exceeding 4 mole percent of the total epoxidation reactor feed. This high carbon dioxide concentration in the epoxidation reactor feed does not normally have a significantly undesirable impact on the catalytic performance characteristics of high activity epoxidation catalysts; however, with the use of a high selectivity epoxidation catalysts, as opposed to the use of the high activity epoxidation catalysts, in an epoxidation process huge process benefits are obtainable with the processing of an epoxidation reactor feed that has a low carbon dioxide concentration relative to conventional ethylene oxide manufacturing processes.

The inventive method provides a way to realize the aforementioned benefits that result from the use of a high selectivity epoxidation catalyst in the processing of an epoxidation reactor feed by providing for the reduction in the carbon dioxide concentration of the epoxidation reactor feed.

To recover the ethylene oxide as a product, the epoxidation reactor effluent from the epoxidation reactor is charged to an ethylene oxide absorber system. The ethylene oxide absorber system can be any system or means known to those skilled in the art that defines ethylene oxide separation zone and provides means for separating the ethylene oxide from the epoxidation reactor effluent and to yield an ethylene oxide stream, comprising ethylene oxide, and a gaseous overhead stream, comprising unreacted ethylene, unreacted oxygen, and carbon dioxide. The gaseous overhead stream can further comprise inert compounds.

To remove carbon dioxide from the ethylene oxide manufacturing process system and to achieve the reduced carbon dioxide concentration levels in the epoxidation reactor feed, the gaseous overhead stream may be divided into a split portion stream and a remaining portion stream. The remaining portion stream is charged to the carbon dioxide removal system, and the split portion stream, if any, is optionally recycled back to the epoxidation reactor.

The remaining portion stream includes at least 40 mole percent of the total gaseous overhead stream upwardly to 100 mole percent of the gaseous overhead stream. The molar ratio of the moles of the remaining portion stream to the moles of the gaseous overhead stream is in the range of from 0.4 upwardly to 1 and, preferably, this ratio is in the range of from 0.5 to 1. In one particular and especially preferred embodiment of the inventive method it is desirable for substantially the entire gaseous overhead stream to be charged to the carbon dioxide removal system. What is meant by substantially is that either all, that is, 100 mole percent, or at least 80 or 90 mole percent of the total gaseous overhead stream is passed and charged to the carbon dioxide removal system.

The carbon dioxide removal system can be any system or means known to those skilled in the art that defines a separation zone and provides means for separating the gaseous overhead stream into a carbon dioxide depleted gas stream and a carbon dioxide gas stream. The carbon dioxide gas stream is the vent stream for the system and comprises carbon dioxide.

In order to provide for the low concentration of carbon dioxide in the epoxidation reactor feed, it is important for the carbon dioxide concentration of the carbon dioxide depleted gas stream to be as low as is feasible, which can be less than 3 mole percent carbon dioxide, relative to the total carbon dioxide depleted gas stream. It is preferable, however, for the carbon dioxide concentration of the carbon dioxide depleted gas stream to be less than 2 mole percent and, it is most preferable for the carbon dioxide concentration to be less than 1.5 mole percent. A typical range for the carbon dioxide concentration is from 0.1 mole percent to 3 mole percent, or from 0.15 to 2 mole percent, or from 0.2 to 1.5.

The large proportion of the gaseous overhead stream that is charged to the carbon dioxide removal system provides for a significantly reduced concentration of carbon dioxide in the epoxidation reactor feed thereby allowing for the realization of the benefits associated with the processing of an epoxidation reactor feed having a low carbon dioxide concentration with the use of a high selectivity epoxidation catalyst.

The epoxidation reactor feed is contacted, under suitable epoxidation reaction conditions, with a high selectivity epoxidation catalyst as defined herein. The high selectivity epoxidation catalyst, in most instances, is contained within an epoxidation reaction zone defined by an epoxidation reactor that provides means for contacting the epoxidation reactor feed with the high selectivity epoxidation catalyst under such suitable epoxidation reaction conditions.

The epoxidation reactor feed of the inventive method comprises ethylene, oxygen and a concentration of carbon dioxide. The epoxidation reactor feed can also comprise additional other compounds, for example, argon, or nitrogen, or methane, or ethane, or some combination of such other compounds. Typically, the amount of ethylene in the epoxidation reactor feed can be in the range of from 1 to 40 mole percent, the amount of oxygen in the epoxidation reactor feed, excluding nitrogen, can be in the range of from 3 to 12 mole percent, the amount of other compounds in the epoxidation reactor feed can be in the range upwardly to 3 mole percent, with the values of mole percent being based on the total moles of the epoxidation reactor feed.

According to the inventive method the epoxidation reactor feed that is contacted with the high selectivity epoxidation catalyst has a low concentration of carbon dioxide which is less than 2 mole percent of the total moles of epoxidation reactor feed. Preferably, the carbon dioxide concentration of the epoxidation reactor feed is maintained below 1.5 or 1.25 mole percent and, most preferably, the carbon dioxide concentration is below 1 mole percent. The concentration of carbon dioxide in the epoxidation reactor feed is at least 0.1 mole percent, typically at least 0.2 mole percent of the total moles of epoxidation reactor feed.

Suitable epoxidation reaction conditions of the inventive method can include an epoxidation reaction zone temperature maintained during the contacting of the high selectivity epoxidation catalyst with the epoxidation reactor feed that is in the range of from 180 °C to 320 °C. A preferred epoxidation reaction zone temperature is in the range of from 190 °C to 310 °C and, most preferred, the epoxidation reaction zone temperature is in the range of from 200 °C to 300 °C. The gaseous hourly space velocity is generally in the range of from 1500 Nl/(l.h) to 10,000 Nl/(l.h), and the epoxidation reaction zone inlet pressure is generally in the range upwardly to 35 bar, preferably from 5 to 30 bar.

One of the features of the inventive method is that it provides for an epoxidation reaction using the high selectivity epoxidation catalyst at a low epoxidation reaction temperature without the loss in catalyst selectivity, but, in fact, an improvement in catalyst selectivity is achieved with the inventive method. The invention, thus, can provide for a catalyst selectivity exceeding 82 mole percent for an epoxidation reaction temperature that is below 260 °C, and it can even provide for a catalyst selectivity exceeding 85 mole percent for an epoxidation reaction temperature that is below 250 °C. Preferably, the inventive method provides for an epoxidation reaction temperature of less than 240 °C for a catalyst selectivity exceeding 88 mole percent.

As used herein, the term "selectivity" means the mole percent of the desired ethylene oxide formed relative to the total ethylene converted at a given work rate. The term "work rate" for a given catalyst defined to mean the amount of ethylene oxide produced per unit volume of catalyst (e.g., kg per m³) per hour. As it is used herein with reference to the activity of a catalyst, the term "activity" means the temperature required by a catalyst to provide for a given work rate.

Thus, a high activity epoxidation catalyst is a catalyst that employs a lower reaction temperature for a given ethylene oxide yield per quantity of epoxidation catalyst when compared to an alternative epoxidation catalyst. And, a high selectivity epoxidation catalyst is a catalyst, that for a given temperature, provides for a greater percentage of a converted feed that is converted to ethylene oxide product than an alternative epoxidation catalyst.

The high selectivity catalyst referred to herein is a supported silver-based catalyst. The material of the supported silver-based catalyst can be selected from a wide range of porous support materials particularly those which are considered to be inert in the presence of the ethylene oxidation feeds, products and reaction conditions. Such materials can be natural or artificial, and they can include the aluminum oxides, magnesia, zirconia, silica, silicon carbide, clays, pumice, zeolites and charcoal. Alpha alumina is a preferred material for use as the main ingredient of the porous support.

The support material is porous and preferably has a surface area, as measured by the B.E.T. method, of less than 20 m²/g and more in particular from 0.05 to 20 m²/g. Preferably the B.E.T. surface area of the support is in the range of from 0.1 to 10, more preferably from 0.1 to 3.0 m²/g. The B.E.T. method of measuring the surface area has been described in detail by Brunauer, Emmet and Teller in J. Am. Chem. Soc. 60 (1938) 309-316.

It is desirable for the highly selective supported silver-based catalyst of the invention to be one which has an initial selectivity of at least 85%, preferably at least 86% and, most preferably, at least 87%.

The term "initial selectivity" referred to herein means the selectivity of the given catalyst when it is fresh and unused. This recognizes that a catalyst can lose activity with use. The initial selectivity of a given catalyst is determined by measuring the selectivity of the catalyst using a standard testing procedure. In this standard testing procedure, a crushed catalyst (1.27-1.81 mm particle size, i.e. 14-20 mesh sieve fraction) is placed within the 6.35 mm (1/4 inch) diameter stainless steel U-tube of a micro-reactor operated under certain specified process conditions. A standard feed of 30 mole percent ethylene, 7 mole percent carbon dioxide, 8.5 mole percent oxygen, and 54.5 mole percent nitrogen is introduced into the micro-reactor at a pressure of 1447 kPa gauge (210 psig) and at such a rate as to provide a gaseous hourly space velocity of 3300 Nl/(l.h). The selectivity, Sw, and activity, Tw, are determined for a work rate of 200 kg ethylene oxide yield per hour per cubic meter of catalyst. The selectivity is presented in terms of mole percent, and the activity is presented in terms of temperature in degrees centigrade.

The high selectivity catalysts of the invention include a rhenium promoter component. Also, in addition to the rhenium component, the high selectivity catalysts can further contain a promoting amount of an alkali metal promoter or a further metal promoter, or both. Suitable high selectivity catalysts are described in detail in U.S. Patents 4,761,394 and 4,766,105, which are incorporated herein by reference.

The high selectivity catalysts, thus, comprise a support material, a catalytically effective amount of silver, a promoting amount of rhenium and, optionally, a promoting amount of one or more alkali metals and, optionally, a promoting amount of one or more additional promoter metals. The amount of silver in the high selectivity catalyst can be in the range of from a catalytically effective amount upwardly to 40 percent by weight of the total catalyst. Preferably, the amount of silver can range from 1 to 30 weight percent based on the total weight of the catalyst and, most preferably, from 5 to 20 weight percent.

The amount of rhenium in the high selectivity catalyst is a promoting amount generally ranging from a promoting amount upwardly to 20 micromoles of rhenium per gram of catalyst. The preferred amount of rhenium in the high selectivity catalyst ranges from 0.1 micromoles per gram to 10 micromoles per gram, more preferably from 0.2 micromoles per gram to 5 micromoles per gram of total catalyst, or, alternatively stated, from 19 parts per million to 1860 parts per million, preferably from 37 parts per million to 930 parts per million by weight of total catalyst.

The amount of alkali metal in the high selectivity catalyst, if any, is a promoting amount, generally ranging from a promoting amount upwardly to 4000 parts per million by weight of the total catalyst (ppmw). Preferably, the amount of alkali metal, when present, is in the range of from 10 to 3000 ppmw, more preferably, from 15 to 2000 ppmw and, even more preferably, from 20 to 1500 ppmw.

The optional additional metal promoter of the high selectivity catalyst can be selected from the group of metals consisting of sulfur, molybdenum, tungsten, chromium, and mixtures of two or more thereof. The amount of additional metal promoters in the high selectivity catalyst, if any, is generally in the range of from 0.1 to 10 millimoles per kilogram of total catalyst, and, preferably, from 0.2 to 5 millimoles per kilogram of total catalyst.

Now referring to FIG. 1, wherein is presented a schematic representation of a typical ethylene oxide manufacturing process system 10 for use in this invention, which includes epoxidation reactor system 12, ethylene oxide recovery system or ethylene oxide absorber 14, and carbon dioxide removal system 16. Epoxidation reactor system 12 includes epoxidation reactor 18, which provides means for contacting a feed stream containing oxygen, ethylene and a first concentration of carbon dioxide with an epoxidation catalyst under suitable epoxidation reaction conditions to thereby yield ethylene oxide. Epoxidation reactor 18 defines an epoxidation reaction zone and contains a volume of high selectivity epoxidation catalyst.

The carbon dioxide removal system 16 includes carbon dioxide absorber 20 and solvent regenerator 22. Carbon dioxide absorber 20 defines a carbon dioxide absorption zone and provides means for receiving a carbon dioxide containing gaseous feed with a lean solvent so as to yield a rich solvent, containing carbon dioxide, and a carbon dioxide depleted gas stream. Solvent regenerator 22 defines a solvent regeneration zone and provides means for separating carbon dioxide from the rich solvent so as to yield a carbon dioxide stream and the lean solvent, which is used as a feed to carbon dioxide absorber 20.

In the operation of an ethylene oxide manufacturing process system 10 a reactor feed, having a first concentration of carbon dioxide, is charged to epoxidation reactor 18 through conduit 24 where within epoxidation reactor 18 the reactor feed is contacted under suitable epoxidation reaction conditions with the high selectivity epoxidation catalyst.

An epoxidation reactor effluent is yielded from epoxidation reactor 18 of the epoxidation reactor system 12 and is charged to ethylene oxide absorber 14 via conduit 26. Ethylene oxide absorber 14 defines an ethylene oxide absorption zone and provides separation means and means for contacting an absorption solvent, such as water, with the epoxidation reactor effluent and to yield a gaseous overhead stream and an ethylene oxide stream. The absorption solvent is introduced into ethylene oxide absorber 14 by way of conduit 28 where within ethylene oxide absorber 14 it is contacted with the epoxidation reactor effluent. An ethylene oxide stream, comprising the absorption solvent and ethylene oxide, passes from ethylene oxide absorber 14 by way of conduit 30 and the gaseous overhead stream, having a second carbon dioxide concentration, passes from ethylene oxide absorber 14 by way of conduit 32 to recycle compressor 34. In addition to the carbon dioxide contained in the gaseous overhead stream it also can further comprise ethylene, or oxygen, or inert compounds, or any combination thereof.

Recycle compressor 34 defines a compression zone and provides means for compressing the gaseous overhead stream. The discharge of the compressed gaseous overhead stream passes from recycle compressor 34 through conduit 36. A split portion, if any, of the compressed gaseous overhead stream, if any, passes by way of conduit 38 and then conduit 40 where it is combined with oxygen introduced by way of conduit 42 and ethylene introduced by way of conduit 44.

It is a feature of the inventive method for the remaining portion stream of the gaseous overhead stream to be at least 30 percent, preferably at least 40 percent and, most preferably, at least 50 percent to 100 percent of the compressed gaseous overhead stream. This remaining portion stream is charged to carbon dioxide absorber 20 of carbon dioxide removal system 16 by way of conduit 46 as the carbon dioxide containing gaseous feed. Yielded by way of conduit 48, is carbon dioxide depleted gas stream, having a third carbon dioxide concentration. The carbon dioxide depleted gas stream passes to conduit 40 where it is combined with oxygen and ethylene respectively introduced into conduit 40 through conduits 42 and 44 and, optionally, the split portion stream. The combination of these streams form the reactor feed charged to epoxidation reactor 18 by way of conduit 24. A carbon dioxide gas stream, comprising carbon dioxide, is yielded as the carbon dioxide stream from solvent regenerator 22 of the carbon dioxide removal system 16 through conduit 50.

The ethylene oxide produced by the inventive method may be converted into 1,2-ethanediol, a 1,2-ethanediol ether, or an ethanolamine. As this invention leads to a more attractive process for the manufacture of ethylene oxide, it concurrently leads to a more attractive process which comprises producing ethylene oxide in accordance with the invention and the subsequent use of the obtained ethylene oxide in the manufacture of the 1,2-ethanediol, 1,2-ethanediol ether, and/or ethanolamine.

The conversion into 1,2-ethanediol or the 1,2-ethanediol ether may comprise, for example, reacting the ethylene oxide with water, suitably using an acidic or a basic catalyst. For example, for making predominantly 1,2-ethanediol and less 1,2-ethanediol ether, the ethylene oxide may be reacted with a ten fold molar excess of water, in a liquid phase reaction in presence of an acid catalyst, e.g. 0.5-1.0 %w sulfuric acid, based on the total reaction mixture, at 50-70 °C at 1 bar absolute, or in a gas phase reaction at 130-240 °C and 20-40 bar absolute, preferably in the absence of a catalyst. If the proportion of water is lowered the proportion of 1,2-ethanediol ethers in the reaction mixture is increased. The 1,2-ethanediol ethers thus produced may be a di-ether, triether, tetra-ether or a subsequent ether. Alternative 1,2-ethanediol ethers may be prepared by converting the ethylene oxide with an alcohol, in particular a primary alcohol, such as methanol or ethanol, by replacing at least a portion of the water by the alcohol.

The conversion into the ethanolamine may comprise, for example, reacting the ethylene oxide with ammonia. Anhydrous or aqueous ammonia may be used, although anhydrous ammonia is typically used to favour the production of monoethanolamine. For methods applicable in the conversion of the ethylene oxide into the ethanolamine, reference may be made to, for example US-A-4845296, which is incorporated herein by reference.

The 1,2-ethanediol and the 1,2-ethanediol ether may be used in a large variety of industrial applications, for example in the fields of food, beverages, tobacco, cosmetics, thermoplastic polymers, curable resin systems, detergents, heat transfer systems, etc. The ethanolamine may be used, for example, in the treating ("sweetening") of natural gas.

Unless specified otherwise, the low-molecular weight organic compounds mentioned herein, for example the 1,2-ethanediol ethers and reaction modifiers, have typically at most 40 carbon atoms, more typically at most 20 carbon atoms, in particular at most 10 carbon atoms, more in particular at most 6 carbon atoms. As defined herein, ranges for numbers of carbon atoms (i.e. carbon number) include the numbers specified for the limits of the ranges.

Having generally described the invention, a further understanding may be obtained by reference to the following examples, which are provided for purposes of illustration only and are not intended to be limiting unless otherwise specified.

### EXAMPLE 1

A high selectivity catalyst, containing silver and promoting amounts of rhenium, lithium, cesium and sulfur on alpha-alumina was tested in the production of ethylene oxide from ethylene and oxygen. To do this, a sample of crushed catalyst was loaded into a stainless steel U-shaped reactor tube. The tube was immersed in a molten metal bath (heat medium) at 180 °C, and the ends of the tube were connected to a gas flow system. A gas mixture passed through the catalyst bed, in a "once-through" operation. The weight of catalyst used and the inlet gas flow rate were adjusted to give a gas hourly space velocity of 3300 Nl/(l.h). The inlet gas pressure was 1550 kPa absolute.

The composition of the gas mixture was adjusted to 30 volume percent ethylene, 8 volume percent oxygen, 1 volume percent carbon dioxide, 2.5 parts per million by volume (ppmv) ethyl chloride, and nitrogen balance.

The temperature of the catalyst bed was ramped up at a rate of 10 °C per hour to 225 °C and then the temperature was adjusted so as to achieve an oxygen conversion of 40 mole percent. The ethyl chloride concentration in the gas mixture was adjusted to 2.5 ppmv so as to obtain an optimum selectivity of ethylene oxide formation. The activity of the catalyst is expressed as the temperature at which a 40 mole percent oxygen conversion is achieved (T40); the selectivity is the selectivity at the temperature T40. During the run the catalyst was subject to degradation, and in order to maintain a constant 40 mole percent oxygen conversion the temperature was gradually increased. The results are given in TABLE 1.

In three similar comparative tests, the concentration of carbon dioxide in the gas mixture was 5 to 7 percent volume, instead of 1 percent volume. The average result of the three comparative tests is also given in TABLE 1.

**TABLE 1**

| CO₂ concentration, %v | 1 | 5-7 |
|---|---|---|
| Run time, days | 263 | 195 |
| T40, initial, °C | 248 | 261 |
| Average activity decline rate, °C/month | 2.1 | 2.9 |
| Initial selectivity, %mole | 86.0 | 85.1 |
| Average selectivity decline rate, %mole/month | 0.7 | 1.1 |
| T40: temperature at 40 %mole oxygen conversion | | |

The results in TABLE 1 show clearly that a lower carbon dioxide concentration in the epoxidation reactor feed improves the performance of a high selectivity catalyst, in respect of its activity, selectivity and catalyst life.

### EXAMPLE 2

This calculated example presents data generated by a proprietary model for predicting the performance of a high selectivity epoxidation catalyst at the operating conditions of an hourly space velocity of 4700 Nl/(l.h), a pressure of 21.7 barg, and a work rate of 184 kg/(m³.h) for a reactor feed containing 25 mole percent ethylene and 8 mole percent oxygen. The model is based on the correlation of actual catalyst performance data gathered from numerous sources such as micro-reactor activity data, pilot plant data and other sources of catalyst performance data.

FIG. 2 presents the selectivity of a high selectivity epoxidation catalyst as a function of the age of the catalyst based on the cumulative ethylene oxide production in kton/m³ for the corresponding feedstock carbon dioxide concentrations presented in FIG. 4. The plots show that there is a strong relationship between catalyst life and feedstock carbon dioxide concentration and between selectivity and feedstock carbon dioxide concentration. As is shown in FIG. 2, the rate of decline in the selectivity of the catalyst when processing a feedstock having a carbon dioxide concentration of less than 1 mole percent (curve marked "I") is significantly lower than the rate of decline in the selectivity of the catalyst when processing a feedstock having a more conventional carbon dioxide concentration of greater than 4 mole percent (curve marked "II"). It is also noted that the initial selectivity of the high selectivity catalyst is higher for the case in which the feedstock has a carbon dioxide concentration of less than 1 mole percent as opposed to a feedstock carbon dioxide concentration of greater than 4 mole percent. The feedstock having a carbon dioxide concentration of less than 1 mole percent is obtained by charging substantially all gaseous overhead stream as the carbon dioxide containing feed gas to the carbon dioxide removal system means for separating said carbon dioxide containing feed gas.

These data demonstrate the great benefits in the selectivity and life of a high selectivity epoxidation catalyst that are obtainable from processing an epoxidation reactor feedstock having a low carbon dioxide concentration. Further comparative data relate to the use of a high activity catalyst operated at greater than 4 mole percent carbon dioxide concentration (curve marked "III").

FIG. 3 presents the reactor coolant temperature as a function of the age of the catalyst used in the epoxidation reaction for the corresponding feedstock carbon dioxide concentrations presented in FIG. 4. The reactor coolant temperature approximates the reaction temperature. As the data demonstrate, the epoxidation catalyst of the inventive method that processes an epoxidation reactor feedstock having a low carbon dioxide concentration of less than 1 mole percent (curve marked "I") loses its activity at significantly lower rate than the epoxidation catalyst of the conventional method that processes an epoxidation reactor feedstock having a significantly higher concentration of carbon dioxide than that of the inventive method (curve marked "II"). These data show that the stability of the high selectivity epoxidation catalyst in terms of the rate of decline in catalyst activity is significantly improved with the inventive method which includes the processing of an epoxidation feedstock having a very low carbon dioxide concentration. Further comparative data relate to the use of a high activity catalyst operated at greater than 4 mole percent carbon dioxide concentration (curve marked "III").

## Claims

1. A method of manufacturing ethylene oxide, said method comprises:
charging a reactor feed comprising ethylene, oxygen, and a concentration of carbon dioxide to an epoxidation reaction zone containing a high selectivity supported silver-based epoxidation catalyst comprising a promoting amount of rhenium and operated under epoxidation reaction conditions, wherein said concentration of carbon dioxide is in the range of from 0.1 to less than 2 mole percent, of the total reactor feed;
yielding from said epoxidation reaction zone an epoxidation reactor effluent;
charging at least a portion of said epoxidation reactor effluent to an ethylene oxide absorber means for separating said at least a portion of said epoxidation reactor effluent into a gaseous overhead stream and an ethylene oxide stream;
dividing said gaseous overhead stream into a split portion stream, if any, and a remaining portion stream wherein said remaining portion stream is at least 40 percent upwardly to 100 percent of said gaseous overhead stream;
charging said remaining portion stream as a carbon dioxide containing feed gas to a carbon dioxide removal system means for separating said carbon dioxide containing feed gas into a carbon dioxide depleted gas stream and a carbon dioxide gas stream; and
combining at least a portion of said carbon dioxide depleted gas stream with oxygen and ethylene to thereby form said reactor feed.

2. A method as recited in claim 1, wherein said combining step further includes combining at least a portion of said split portion stream with said at least a portion of said carbon dioxide depleted gas stream, said oxygen, and said ethylene to thereby form said reactor feed.

3. A method as recited in claim 1 or 2, wherein said epoxidation reaction conditions include an epoxidation reaction zone temperature below 260 °C.

4. A method as recited in claim 3, wherein said epoxidation reaction conditions include an epoxidation reaction zone temperature below 250 °C, in particular in the range of from 180 to 250 °C, more in particular in the range of from 190 to 240 °C.

5. A method as recited in any of claims 1-4, wherein said concentration of carbon dioxide is in the range of from 0.2 to less than 1.25 mole percent, of the total reactor feed.

6. A method as recited in any of claims 1-5, wherein said remaining portion stream is at least 50 percent upwardly to 100 percent of said gaseous overhead stream.

7. A method as recited in claim 6, wherein substantially all said gaseous overhead stream is charged as the carbon dioxide containing feed gas to the carbon dioxide removal system means for separating said carbon dioxide containing feed gas.

8. A method as recited in any of claims 1-7, wherein said high selectivity supported silver-based epoxidation catalyst comprises a support material, a catalytically effective amount of silver, and a promoting amount of rhenium.

9. A method as recited in claim 8, wherein the support material is an alpha alumina, the amount of silver is in the range of from 1 to 40 weight percent, and the amount of rhenium is in the range of from 0.1 to 10 micromoles per gram, based on the total weight of catalyst.

10. A method for making 1,2-ethanediol or a 1,2-ethanediol ether comprising:
- obtaining ethylene oxide by a method of manufacturing ethylene oxide as recited in any of claims 1-9, and
- converting ethylene oxide into 1,2-ethanediol or the 1,2-ethanediol ether.

## Patentansprüche

1. Verfahren zum Herstellen von Ethylenoxid, wobei das Verfahren umfasst:
Beladen einer Epoxidierungsreaktionszone enthaltend einen hochselektiven, geträgerten, Silber basierenden Epoxidierungskatalysator umfassend eine Promotormenge an Rhenium und betrieben unter Epoxidierungsreaktionsbedingungen, mit einem Reaktorzulauf umfassend Ethylen, Sauerstoff, und eine Konzentration an Kohlenstoffdioxid, wobei die Konzentration an Kohlenstoffdioxid in dem Bereich von 0,1 bis weniger als 2 Molprozent des gesamten Reaktorzulaufs liegt;
Erhalten eines Epoxidierungsreaktorablaufs aus der Epoxidierungsreaktionszone;
Beladen eines Ethylenoxidabsorbermittels mit mindestens einem Teil des Epoxidierungsreaktorablaufs zum Trennen des mindestens einen Teils des Epoxidierungsreaktorablaufs in einen gasförmigen Kopfstrom und einen Ethylenoxidstrom;
Teilen des gasförmigen Kopfstroms in einen abgespaltenen Teilstrom, falls vorhanden, und einen verbleibenden Teilstrom, wobei der verbleibende Teilstrom mindestens 40 % aufwärts bis 100 % des gasförmigen Kopfstroms ausmacht;
Beladen eines Kohlenstoffdioxidentfernungssystemmittels mit dem verbleibenden Teilstrom als ein Kohlenstoffdioxid enthaltendes Zulaufgas zum Trennen des Kohlenstoffdioxid enthaltenden Zulaufgases in einen an Kohlenstoffdioxid verarmten Gasstrom und einen Kohlenstoffdioxidgasstrom; und
Kombinieren von mindestens einem Teil des an Kohlenstoffdioxid verarmten Gasstroms mit Sauerstoff und Ethylen, um **dadurch** den Reaktorzulauf zu bilden.

2. Verfahren wie zitiert in Anspruch 1, wobei der Kombinierschritt ferner beinhaltet Kombinieren von mindestens einem Teil des abgespaltenen Teilstroms mit dem mindestens einen Teil des an Kohlenstoffdioxid verarmten Gasstroms, dem Sauerstoff und dem Ethylen, um **dadurch** den Reaktorzulauf zu bilden.

3. Verfahren wie zitiert in Anspruch 1 oder 2, wobei die Epoxidierungsreaktionsbedingungen eine Epoxidierungsreaktionszonentemperatur von unter 260°C beinhalten.

4. Verfahren wie zitiert in Anspruch 3, wobei die Epoxidierungsreaktionsbedingungen eine Epoxidierungsreaktionszonentemperatur von unter 250°C, insbesondere in dem Bereich von 180 bis 250°C, ganz insbesondere in dem Bereich von 190 bis 240°C, beinhalten.

5. Verfahren wie zitiert in einem der Ansprüche 1-4, wobei die Konzentration an Kohlenstoffdioxid in dem Bereich von 0,2 bis weniger als 1,25 Molprozent des gesamten Epoxidierungsreaktorzulaufs liegt.

6. Verfahren wie zitiert in einem der Ansprüche 1-5, wobei der verbleibende Teilstrom mindestens 50 Prozent aufwärts bis 100 Prozent des gasförmigen Kopfstroms ausmacht.

7. Verfahren wie zitiert in Anspruch 6, wobei das Kohlenstoffdioxidentfernungssystemmittel zum Trennen des Kohlenstoffdioxid enthaltenden Zulaufgases mit im Wesentlichen dem gesamten gasförmigen Kopfstrom als Kohlenstoffdioxid enthaltendes Zulaufgas beladen wird.

8. Verfahren wie zitiert in einem der Ansprüche 1 - 7, wobei der hochselektive, geträgerte, Silber basierende Epoxidierungskatalysator ein Trägermaterial, eine katalytisch effektive Menge an Silber und eine Promotormenge an Rhenium umfasst.

9. Verfahren wie zitiert in Anspruch 8, wobei das Trägermaterial ein alpha-Aluminiumoxid ist, die Menge an Silber in dem Bereich von 1 bis 40 Gewichtsprozent liegt, und die Menge an Rhenium in dem Bereich von 0,1 bis 10 Mikromolen pro Gramm liegt, basierend auf dem gesamten Gewicht des Katalysators.

10. Verfahren zum Herstellen von 1,2-Ethandiol oder eines 1,2-Ethandiolethers umfassend:
- Erhalten von Ethylenoxid durch ein Verfahren zum Herstellen von Ethylenoxid wie zitiert in einem der Ansprüche 1 - 9, und
- Konvertieren von Ethylenoxid in 1,2-Ethandiol oder den 1,2-Ethandiolether.

## Revendications

1. Procédé de fabrication d'oxyde d'éthylène, ledit procédé comprenant les étapes consistant à :
charger une charge d'alimentation de réacteur comprenant de l'éthylène, de l'oxygène et une concentration de dioxyde de carbone dans une zone de réaction d'époxydation contenant un catalyseur d'époxydation à base d'argent supporté à haute sélectivité comprenant une quantité promotrice de rhénium et fonctionnant dans des conditions de réaction d'époxydation, dans lequel ladite concentration de dioxyde de carbone se situe dans la plage allant de 0,1 à moins de 2 moles pour cent de la charge d'alimentation totale du réacteur ;
produire à partir de ladite zone de réaction d'époxydation un effluent de réacteur d'époxydation ;
charger au moins une partie dudit effluent de réacteur d'époxydation dans un moyen formant absorbeur d'oxyde d'éthylène destiné à séparer ladite au moins une partie dudit effluent de réacteur d'époxydation en un courant de tête gazeux et un courant d'oxyde d'éthylène ;
diviser ledit courant de tête gazeux en un courant de partie séparée, le cas échéant, et un courant de partie restante, dans lequel ledit courant de partie restante représente au moins 40 pour cent et jusqu'à 100 pour cent dudit courant de tête gazeux ;
charger ledit courant de partie restante en tant que gaz d'alimentation contenant du dioxyde de carbone dans un moyen formant système d'élimination de dioxyde de carbone destiné à séparer ledit gaz d'alimentation contenant du dioxyde de carbone en un courant gazeux appauvri en dioxyde de carbone et un courant gazeux à dioxyde de carbone ; et
combiner au moins une partie dudit courant gazeux appauvri en dioxyde de carbone avec de l'oxygène et de l'éthylène pour former ainsi ladite charge d'alimentation du réacteur.

2. Procédé selon la revendication 1, dans lequel ladite étape de combinaison comprend en outre la combinaison d'au moins une partie dudit courant de partie séparée avec ladite au moins une partie dudit courant gazeux appauvri en dioxyde de carbone, ledit oxygène et ledit éthylène pour former ainsi ladite charge d'alimentation du réacteur.

3. Procédé selon la revendication 1 ou 2, dans lequel lesdites conditions de réaction d'époxydation comprennent une température de la zone de réaction d'époxydation inférieure à 260 °C.

4. Procédé selon la revendication 3, dans lequel lesdites conditions de réaction d'époxydation comprennent une température de la zone de réaction d'époxydation inférieure à 250 °C, en particulier comprise dans la plage allant de 180 à 250 °C, plus particulièrement dans la plage allant de 190 à 240 °C.

5. Procédé selon l'une quelconque des revendications 1 à 4, dans lequel ladite concentration de dioxyde de carbone se situe dans la plage allant de 0,2 à moins de 1,25 mole pour cent de la charge totale d'alimentation du réacteur.

6. Procédé selon l'une quelconque des revendications 1 à 5, dans lequel ledit courant de partie restante représente au moins 50 pour cent et jusqu'à 100 pour cent dudit courant gazeux de tête.

7. Procédé selon la revendication 6, dans lequel sensiblement tout ledit courant gazeux de tête est chargé en tant que gaz d'alimentation contenant du dioxyde de carbone dans le moyen formant système d'élimination de dioxyde de carbone destiné à séparer ledit gaz d'alimentation contenant du dioxyde de carbone.

8. Procédé selon l'une quelconque des revendications 1 à 7, dans lequel ledit catalyseur d'époxydation à base d'argent supporté à haute sélectivité comprend un matériau de support, une quantité catalytiquement efficace d'argent et une quantité promotrice de rhénium.

9. Procédé selon la revendication 8, dans lequel le matériau de support est une alumine-alpha, la quantité d'argent se situe dans la plage allant de 1 à 40 pour cent en poids, et la quantité de rhénium se situe dans la plage allant de 0,1 à 10 micromoles par gramme, sur la base du poids total de catalyseur.

10. Procédé de fabrication de 1,2-éthanediol ou d'un éther de 1,2-éthanediol comprenant les étapes consistant à :
- obtenir de l'oxyde d'éthylène au moyen d'un procédé de fabrication d'oxyde d'éthylène selon l'une quelconque des revendications 1 à 9, et
- convertir l'oxyde d'éthylène en 1,2-éthanediol ou en l'éther de 1,2-éthanediol.
